# EUROPÄISCHE PATENTSCHRIFT

(11) **EP 3 383 450 B1**
(45) Veröffentlichungstag und Bekanntmachung des Hinweises auf die Patenterteilung: **25.03.2020**
(21) Anmeldenummer: 16810246.5
(22) Anmeldetag: 01.12.2016
(51) Int. Cl.: A61M 1/14, A61M 1/34, G01N 3/12

(54) **VERFAHREN ZUM TESTEN DER RIGIDITÄT EINES DISPOSABLES**
METHOD FOR TESTING THE RIGIDITY OF A DISPOSABLE
PROCÉDÉ POUR TESTER LA RIGIDITÉ D'UN ARTICLE À USAGE UNIQUE

(30) Priorität: 02.12.2015 DE 102015015636
(43) Veröffentlichungstag der Anmeldung: 10.10.2018
(73) Patentinhaber: Fresenius Medical Care Deutschland GmbH, 61352 Bad Homburg (DE)
(72) Erfinder: PETERS, Arne, 61352 Bad Homburg (DE); WIKTOR, Christoph, 63571 Gelnhausen (DE)
(74) Vertreter: Herrmann, Uwe
(86) Internationale Anmeldenummer: PCT/EP2016/002033
(87) Internationale Veröffentlichungsnummer: WO 2017/092870

(56) Entgegenhaltungen:
- EP-A1- 0 687 474
- DE-C1- 19 757 523
- US-A- 3 916 673
- US-A1- 2014 276 421

## Beschreibung

Die vorliegende Erfindung betrifft ein Verfahren zum Testen der Rigidität eines zur volumetrischen Bilanzierung bestimmten Disposables für ein Blutbehandlungsgerät.

Die vorliegende Erfindung betrifft des Weiteren ein Blutbehandlungsgerät.

Aus dem Stand der Technik ist es bekannt, dass Blutbehandlungsgeräte nicht nur eine Reinigung oder sonstige Behandlung des Blutes, wie beispielsweise die Trennung in verschiedene Bestandteile durchführen, sondern als weitere wesentliche Aufgabe eine Bilanzierung von Flüssigkeitsvolumina vornehmen. Als Beispiele für solche Geräte sind Hämodialysegeräte, Peritonealdialysegeräte, Plasmaseparationsgeräte, Leberunterstützungssysteme und im Rahmen einer Sepsis eingesetzte Geräte zu nennen.

So führen Hämodialysegeräte mittels des Dialysators eine Blutbehandlung durch und mittels eines Bilanziersystems wird eine Quantifizierung der dem Dialysator zu- und abgeführten Dialysierflüssigkeitsvolumina vorgenommen.

Dabei kommen Volumenbilanzsysteme zum Einsatz, die über eine Kammer mit einem festen Volumen verfügen, in der sich eine flexible Membran befindet. Die Membran trennt die Kammer in zwei Bereiche. Wird der eine Bereich mit einer Flüssigkeit gefüllt, wird dasselbe Volumen aus der anderen Kammer verdrängt. Das die Kammer bildende starre Gefäß sorgt für die gewünschte Genauigkeit, da sich dessen Volumen aufgrund der starren Kammerwandungen nicht verändert.

Ein solches Volumenbilanzsystem ist aus der DE 10 2013 019 356 A1 bekannt. Diese Druckschrift offenbart eine Vorrichtung und ein Verfahren zum Bilanzieren von Flüssigkeiten für eine extrakorporale Blutbehandlungsvorrichtung mit einer Blutbehandlungseinheit. Die Bilanziervorrichtung verfügt über eine Bilanziereinheit, die in einem Ausführungsbeispiel eine Bilanzkammer aufweist. Bei der Bilanzkammer handelt es sich um ein volumenstarres Behältnis mit einem ersten Auslass und einem zweiten Auslass an der Oberseite und einem gemeinsamen Einlass an der Unterseite.

Um kompakte, höher integrierte Systeme zu bauen, ist es wünschenswert, vergleichbare Systeme bei einem Disposable, d.h. bei einem Wegwerfartikel zu realisieren.

Das Volumen des Disposables wirkt als Microbatch, d.h. kann zur volumetrischen Bilanzierung herangezogen werden, ohne dass eine eigens für diesen Zweck ausgebildete Bilanzkammer vorgesehen wird. Beispielsweise können die Flusswege einschließlich des Vorfilters einer Kassette, d.h. des Disposables zusammen das Batchvolumen bilden, das zur Bilanzierung dient.

Derartige Disposables bestehen üblicherweise aus Kunststoff.

Um die erforderliche Genauigkeit bei der Bilanzierung bereitstellen zu können, müssen die Wandungen des Disposables eine ausreichende Rigidität, d.h. Starrheit aufweisen. Darunter wird verstanden, dass die Wandungen des Disposables so ausgebildet sind, dass das von diesen begrenzte Volumen konstant ist oder im Bereich bestimmter Grenzen liegt.

Vorteilhaft sind somit Systeme bzw. Disposables, deren Rigidität konstant ist oder im Bereich einer bestimmten, noch tolerierbaren Spanne liegt.

Der vorliegenden Erfindung liegt die Aufgabe zugrunde, ein Verfahren bereitzustellen, mittels dessen die Rigidität von Wandabschnitten eines Disposables oder Teilen von diesem gemessen werden kann.

Diese Aufgabe wird durch ein Verfahren mit den Merkmalen des Anspruchs 1 gelöst.

Danach ist vorgesehen, dass das Verfahren die folgenden Schritte umfasst: Befüllen des Disposables oder eines Teils von diesem mit einer Flüssigkeit, Einschließen der eingefüllten Flüssigkeit, so dass in dem Disposable oder in dem Teil von diesem ein bestimmtes Volumen der Flüssigkeit vorliegt, Zufuhr und/oder Abfuhr eines bestimmten Flüssigkeitsvolumens und Messung der durch die Zufuhr und/oder Abfuhr des genannten Flüssigkeitsvolumens bedingten Druckänderung.

Gemäß der Erfindung wird somit ein bestimmtes Volumen des Disposables, das im Folgenden auch als Kassette bezeichnet wird, okkludiert, d.h. eingeschlossen, was beispielsweise durch Klemmen oder Ventile erfolgen kann. Sodann werden z.B. mittels einer Pumpe ein oder mehrere kurze Pumpstöße ausgeführt. Je rigider die Kassette ist, desto größer fallen die Druckerhöhungen oder -verringerungen in dem durch die Kassette eingeschlossenen Volumen aus, die durch die Pumpstöße hervorgerufen werden.

Ist die Kassette sehr rigide, d.h. volumenkonstant, ergeben sich größere Anstiege oder Abfälle des gemessenen Drucks als bei einer Kassette, deren Wandungen nachgiebig sind und somit eine größere Volumenveränderung zulassen.

Die Messung der Rigidität wird von allen Faktoren beeinflusst, die eine Volumenveränderung nach sich ziehen können, wie beispielsweise das fehlerhafte Vorhandensein von Luft in dem Disposable nach dem Einfüllen der Flüssigkeit. Ergibt der Test eine zu geringe Rigidität, kann als erste Maßnahme zur Abhilfe der Vorgang des Füllens/Spülens wiederholt werden, so dass die Störung durch etwaige Lufteinschlüsse ausgeschlossen werden kann. Wird bei einer oder mehreren Wiederholungen des Test bzw. des erfindungsgemäßen Verfahrens erneut der Grenzwert für die Rigidität nicht erreicht, so kann auf die fehlerhafte Beschaffenheit der Kassettenwand bzw. des Disposables geschlossen werden.

Der Grenzwert für die Rigidität kann so gewählt sein, dass kleine Volumenänderungen durch Lufteinschlüsse oder fehlerhafte Beschaffenheit der Kassettenwand bzw. des Disposables, die die tolerierte Bilanzfehlergrenze nicht überschreiten würden, noch akzeptiert werden. Dies kann beispielsweise der Fall sein, wenn der Fehler bezüglich des gesamten, über die Behandlungsdauer durch die Kassette bzw. das Disposable geleiteten Flüssigkeitsvolumens bei < 10 Promille oder beispielsweise bei < 2 Promille liegt. In diesem Falle kann die Kassette bzw. das Disposable gleichwohl als in Ordnung qualifiziert werden.

Das erfindungsgemäße Verfahren wird vor der Behandlung des Patienten mit dem Blutbehandlungsgerät durchgeführt, vorzugsweise direkt nach dem Befüllen des Disposables. Der Patient steht während der Durchführung des Verfahrens nicht in Fluidverbindung mit dem Blutbehandlungsgerät. Vorzugsweise ist der Patient während des Verfahrens nicht mit dem Blutbehandlungsgerät verbunden.

Von der Erfindung ist auch der Fall umfasst, dass kein Flüssigkeitsvolumen zu- oder abgeführt werden kann, d.h. die Kassette vollständig rigide ist und somit keine Volumenveränderung zulässt.

Um den Test gemäß dem erfindungsgemäßen Verfahren zu bestehen, muss somit die gemessene Druckamplitudenänderung, d.h. der gemessene Druckanstieg oder Druckabfall größer als ein Referenzwert sein. Dieser Referenzwert wurde vorab als für die volumetrische Bilanzierung ausreichender Wert festgelegt.

Unter dem Begriff der "Messung einer Druckänderung" ist auch der Fall zu verstehen, dass nicht die Druckänderung als solche gemessen wird, sondern dass Druckwerte gemessen werden und die Differenz, d.h. die Druckänderung bestimmt wird. Entsprechendes gilt für die Begriffe der Messung eines Druckanstieges oder eines Druckabfalls.

Denkbar ist es, dass die Zufuhr bzw. Abfuhr des Flüssigkeitsvolumens mittels einer oder mehrerer Pumpen erfolgt, die in Fluidverbindung zu dem Disposable stehen, so dass das Flüssigkeitsvolumen dem Innenraum des Disposables zu- oder abgeführt werden kann.

Bei der oder den Pumpen handelt es sich vorzugsweise um Pumpen, die ohnehin Bestandteile eines Blutbehandlungsgerätes bilden, was den Vorteil mit sich bringt, dass keine eigens für die Durchführung des Verfahrens bestimmte Pumpen bereitgestellt werden müssen.

Denkbar ist es, dass es sich bei der Pumpe um die um die Ultrafiltrationspumpe oder um die Dialysatpumpe eines Dialysegerätes handelt.

Die Erfindung ist jedoch nicht auf Dialysegeräte beschränkt. Auch beliebige andere Blutbehandlungsgeräte sind von der Erfindung umfasst, wie z.B. Plasmaseparationsgeräte, Leberunterstützungssysteme, Systeme zur Behandlung einer Sepsis etc.

In einer denkbaren Ausgestaltung der Erfindung wird das Verfahren so durchgeführt, dass die Zufuhr und/oder Abfuhr des weiteren Flüssigkeitsvolumens sowie die Messung des Druckanstieges bzw. des Druckabfalls mehrfach durchgeführt wird.

Dabei ist es denkbar, dass stets dasselbe Flüssigkeitsvolumen oder auch unterschiedliche Flüssigkeitsvolumina zu- bzw. abgeführt werden. Auf diese Weise können Störeffekte vermieden werden.

Das Okkludieren, d.h. das Einschließen der Flüssigkeit kann beispielsweise durch das Schließen von Ventilen oder Klemmen erfolgen. Bei den Ventilen oder Klemmen handelt es sich vorzugsweise um die des Blutbehandlungsgerätes.

In einer weiteren Ausgestaltung der Erfindung ist vorgesehen, dass das Disposable wenigstens zwei Bilanzierkammern aufweist und dass wenigstens ein die Bilanzierkammern verbindender Kanal vorgesehen ist. Somit können durch das erfindungsgemäße Verfahren die im Betrieb voneinander getrennten Kammern gleichzeitig auf ihre Rigidität hin getestet werden.

In einer weiteren Ausgestaltung der Erfindung erfolgt die Druckmessung an der Pumpe oder in einem Bereich des Disposables.

Grundsätzlich kann die Druckmessung an jeder Position erfolgen, an der die Zu- oder Abfuhr des Flüssigkeitsvolumens zu einer Druckänderung führt, die mit der Rigidität des Disposables korreliert.

Die vorliegende Erfindung betrifft des Weiteren ein Blutbehandlungsgerät mit wenigstens einer Aufnahme für ein zur volumetrischen Bilanzierung bestimmtes Disposable, wobei das Blutbehandlungsgerät einen oder mehrere Aktoren aufweist, die dazu ausgebildet sind, auf das Disposable einzuwirken, wobei das Blutbehandlungsgerät eine oder mehrere Pumpen aufweist, die dazu ausgebildet sind, ein bestimmtes Flüssigkeitsvolumen in das und/oder aus dem Disposable zu fördern, und wobei das Blutbehandlungsgerät einen oder mehrere Drucksensoren aufweist, die ausgebildet sind, den Druck in der in dem Disposable befindlichen Flüssigkeit zu messen, wobei das Blutbehandlungsgerät wenigstens einen Prozessor aufweist, der ggf. den oder die Aktoren und die eine oder mehreren Pumpen ansteuert und der programmiert ist, ein Verfahren nach einem der Ansprüche 1 bis 8 durchzuführen.

Wie oben ausgeführt, kann es sich bei dem Blutbehandlungsgerät beispielsweise um ein Dialyse- oder Plasmaseparationsgerät, um ein Leberunterstützungssystem oder auch um ein Gerät zur Behandlung einer Sepsis handeln.

Bei der Pumpe kann es sich um die Ultrafiltrationspumpe oder um die Dialysatpumpe oder auch um eine sonstige Pumpe des Blutbehandlungsgerätes handeln.

Auch der Einsatz einer separaten Pumpe, d.h. einer Pumpe, die keinen Bestandteil des Blutbehandlungsgerätes bildet, ist denkbar und von der Erfindung mit umfasst.

Der zur Erfassung der Druckänderung dienende Drucksensor befindet sich vorzugsweise in oder an der Pumpe oder in oder an dem Disposable.

Denkbar ist es weiterhin, dass das Blutbehandlungsgerät ein oder mehrere Ventile aufweist, mittels derer ein bestimmtes Flüssigkeitsvolumen in dem Disposable absperrbar ist.

In einer weiteren Ausgestaltung der Erfindung weist das Blutbehandlungsgerät eine Auswerteeinheit auf, die ausgebildet ist, den gemessen Druckanstieg bzw. Druckabfall mit einem Grenzwert zu vergleichen.

Dabei ist es des Weiteren denkbar, dass das Blutbehandlungsgerät eine Anzeigeeinheit aufweist, die ausgebildet ist, um anzuzeigen, ob die gemessene Druckänderung einen Grenzwert übersteigt oder nicht.

Alternativ oder zusätzlich kann das Blutbehandlungsgerät eine Sperreinheit aufweisen, die ausgebildet ist, den Betrieb des Blutbehandlungsgerätes zu verhindern, wenn die gemessene Druckänderung den Grenzwert nicht erreicht oder übersteigt.

Auf diese Weise kann eine Blutbehandlung mit einem Disposable verhindert werden, das nicht den Erfordernissen an die gewünschte Rigidität genügt.

Weitere Einzelheiten und Vorteile der Erfindung werden anhand eines in der Zeichnung dargestellten Ausführungsbeispiels näher erläutert.

Es zeigen:
- Figur 1:: eine schematische Ansicht eines Dialysegerätes mit Disposable als Bilanziersystem,
- Figur 2:: eine schematische Ansicht eines Dialysegerätes gemäß Figur 1 mit einer Ultrafiltrationspumpe zur Zufuhr eines weiteren Flüssigkeitsvolumens sowie mit einer Verbindungsleitung zur Verbindung der zu testenden Kanäle,
- Figur 3:: eine weitere schematische Ansicht eines Dialysegerätes gemäß Figur 1 mit einer gegenüber Figur 2 geänderten Anordnung der Pumpe und einer gegenüber Figur 2 geänderten Anordnung der Verbindungsleitung zur Verbindung der zu testenden Kanäle,
- Figur 4:: eine weitere schematische Ansicht eines Dialysegerätes gemäß Figur 1 mit zwei Verbindungsleitungen zur Verbindung der zu testenden Kanäle sowie mit möglichen Anordnungen von Drucksensoren,
- Figur 5:: eine Ansicht des Druckverlaufes über die Zeit bei einer Volumenverschiebung und
- Figur 6:: eine Ansicht des Druckverlaufes über die Zeit bei mehreren aufeinander folgenden Volumenverschiebungen und bei einem Druckhaltetest.

In den Figuren sind gleiche oder funktionsgleiche Teile durch identische Bezugszeichen gekennzeichnet.

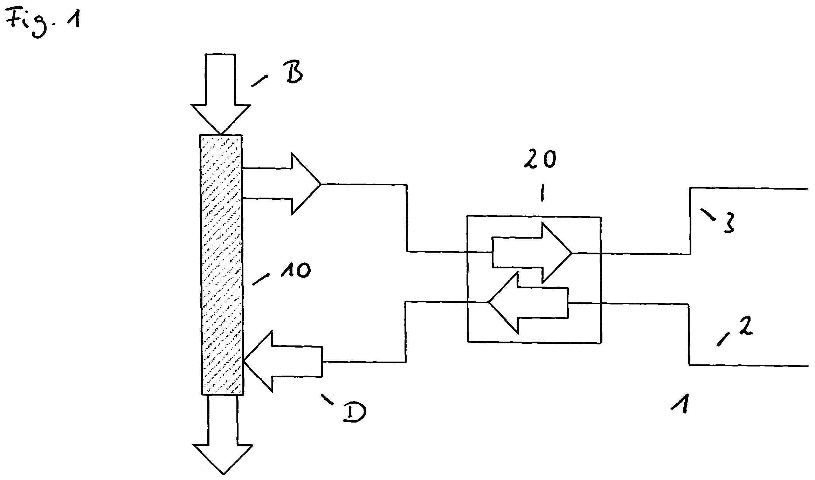

Figur 1 zeigt mit dem Bezugszeichen 1 eine Kassette, die als Disposable ausgeführt ist und die in eine nicht dargestellte Aufnahme eines Dialysegerätes eingesetzt ist.

Von dem Dialysegerät ist mit dem Bezugszeichen 10 der Dialysator dargestellt, dessen Innenraum mittels einer aus einem Hohlfaserbündel bestehenden Membran in eine Dialysatkammer und eine Blutkammer geteilt ist. Die Blutkammer umfasst die Innenräume der Hohlfasern.

Der Blutfluss durch den Dialysator 10 ist mit dem Bezugszeichen B und der Dialysatfluss durch die Kassette 1 und durch den Dialysator 10 ist mit dem Bezugszeichen D gekennzeichnet.

Bei dem Dialysegerät kann es sich beispielsweise um ein Hämodialyse-, Hamofiltrations- oder Hämodiafiltrationsgerät handeln. Von der Erfindung sind jedoch auch beliebige andere Blutbehandlungsgeräte umfasst.

Die Kassette 1 weist Kanäle 2, 3 und Kammern zum Leiten und Bilanzieren der frischen und der verbrauchten Dialysierflüssigkeit auf.

Die zum Bilanzieren dienenden Kammern der Kassette 1 sind gemeinsamen mit dem Bezugszeichen 20 gekennzeichnet. Das Bilanziersystem 20 ist so ausgebildet, dass das aus einer Kammer verdrängte Volumen dem der anderen Kammer zugeführten Volumen an Dialysierflüssigkeit entspricht. Die Betätigung der Kammern erfolgt durch Aktoren des Dialysegerätes. Des Weiteren weist das Dialysegerät Stößel, Klemmen oder sonstige Aktoren auf, die als Ventile dienen und die Kanäle der Kassette zuzusperren vermögen.

Um einen Rigiditätstest gemäß der vorliegenden Erfindung durchzuführen, wird ein Volumen der Kassette 1 durch das Schließen von Ventilen, d.h. beispielsweise durch das Abklemmen von Kanälen der Kassette 1 abgesperrt.

Es wird somit ein bestimmtes Flüssigkeitsvolumen okkludiert.

Beispielsweise werden Ventile in den Zu- und Abführkanälen 2, 3 für die Dialysierflüssigkeit und zwischen dem Bilanziersystem 20 und dem Dialysator 10 sowie auf der von dem Dialysator abgewandten Seite des Bilanziersystems geschlossen, so dass dazwischen befindliche Volumen okkludiert ist.

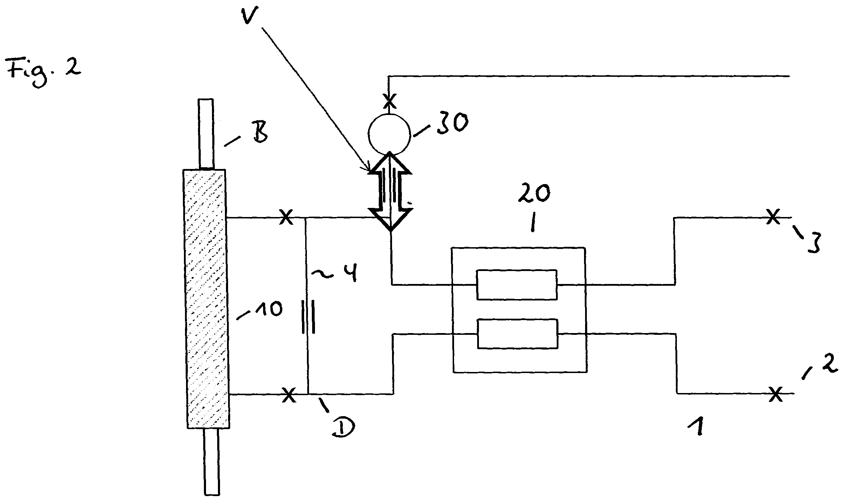

Eine solche Situation ergibt sich aus Figur 2. In Figur 2 sind geschlossene Kanäle der Kassette 1 bzw. geschlossene Ventile durch das Bezugszeichen "x" gekennzeichnet. Ein offener Kanal bzw. ein offenes Ventil ist durch das Bezugszeichen "II" gekennzeichnet. Dies gilt für die weiteren Figuren entsprechend.

Der nicht abgesperrte Kanal bzw. die Verbindungleitung 4 verbindet die Zu- und Abführkanäle 2, 3 miteinander.

Mit dem Bezugszeichen 30 ist die Ultrafiltrationspumpe des Dialysegerätes gekennzeichnet.

Diese steht mit dem Innenraum der Kassette 1 über einen offenen Kanal derart in Verbindung, dass im Betrieb des Dialysegerätes gebrauchte Dialysierflüssigkeit aus der Kassette 1 abgeführt wird.

Zur Durchführung des erfindungsgemäßen Rigiditätstests wird die Ultrafiltrationspumpe 30 dazu genutzt, eine bekannte Volumenverschiebung in dem okkludierten hydraulischen System der Kassette 1 vorzunehmen. Diese Volumenverschiebung kann in einer Zu- und/oder Abfuhr von Flüssigkeit in die Kassette 1 oder aus der Kassette 1 bestehen.

Durch eine Druckmessung im Inneren der Kassette 1 lässt sich auf die Rigidität der Wandungen der Kassette 1 schließen.

Je nachgiebiger die Kassette 1 ist, d.h. desto geringer die Rigidität der Kassette 1 ist, desto geringer sind die Druckänderungen, die durch eine Volumenverschiebung bedingt sind. Je steifer die Kassette 1 ist, d.h. desto höher die Rigidität der Kassette 1 ist, desto größer sind die Druckänderungen, die durch eine Volumenverschiebung bedingt sind.

Die gemessene Druckänderung ist somit ein Maß für die Rigidität des Teils der Kassette 1, in dem sich das okkludierte Volumen befindet.

In dem in Figur 2 skizierten Fall übt die Ultrafiltrationspumpe 30 die Volumenverschiebung aus. Für diesen Zweck ist jedoch auch jede andere Pumpe geeignet, die eine Volumenänderung in der Kassette 1 herbeiführen kann. Dabei kann es sich um eine Pumpe des Dialysegerätes oder auch um eine eigens für die Bestimmung der Rigidität verwendete Pumpe handeln.

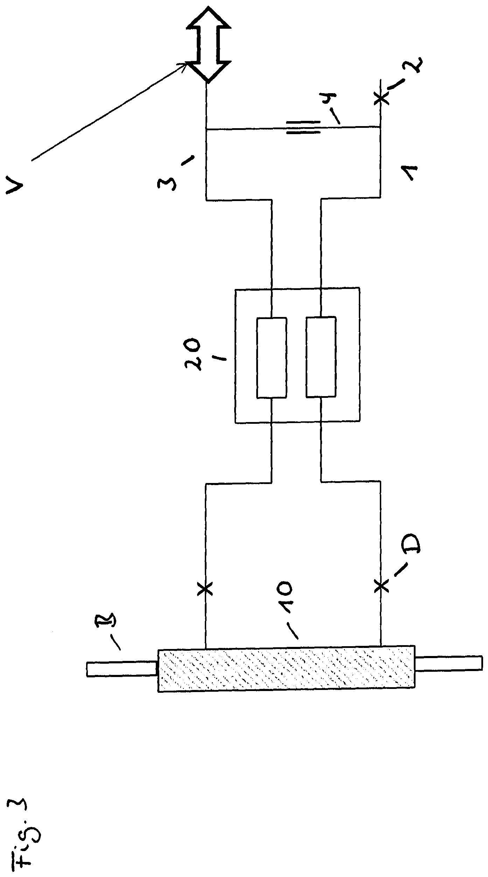

Figur 3 verdeutlicht eine weitere Ausführungsform. Bei dieser befindet sich der die Kanäle 2 und 3 verbindende Kanal 4 nicht zwischen dem Bilanziersystem 20 und dem Dialysator, sondern auf der vom Dialysator abgewandten Seite des Bilanziersystems 20.

Weiter abweichend von der Anordnung gemäß Figur 2 ist der Kanal 3 nur durch ein Ventil abgesperrt. Dieses befindet sich zwischen Bilanziersystem 20 und dem Dialysator 10. Auf der anderen, offenen Seite des Kanals 3 befindet sich eine nicht dargestellte Dialysatpumpe, die die Volumenverschiebung V in der Kassette 1 durchführt.

Ein Vergleich der Figuren 2 und 3 zeigt, dass die Lage der Absperrventile "x" und des offenen Kanals 4 nicht entscheidend ist. Maßgeblich ist vielmehr, dass in Inneren der Kassette 1 ein abgeschlossenes und nur zu der Pumpe hin offenes Flüssigkeitsvolumen gebildet wird, dem durch die Pumpe oder dergleichen ein weiteres Flüssigkeitsvolumen zugeführt wird oder aus dem mittels der Pumpe oder dergleichen ein bestimmtes Volumen abgeführt wird.

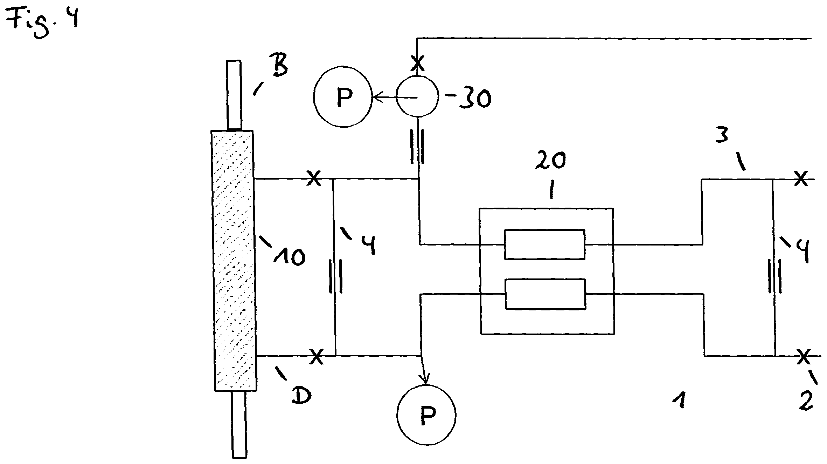

Figur 4 zeigt ein Ausführungsbeispiel, bei dem ein die Kanäle 2 und 3 verbindender Kanal 4 sowohl zwischen dem Bilanziersystem 20 und dem Dialysator 10 als auch auf der vom Dialysator 10 abgewandten Seite des Bilanziersystems 20 angeordnet ist.

Des Weiteren sind mit den Bezugszeichen P Drucksensoren gekennzeichnet, die den Druck in dem okkludierten Volumen der Kassette 1 messen. Wie aus Figur 4 ersichtlich, kann ein solcher Sensor P an oder in der Pumpe 30 oder auch in oder an einem der Kanäle 2, 3 angeordnet sein.

Auch andere Orte der Druckmessung sind denkbar und von der Erfindung umfasst. So ist es beispielsweise möglich, den Druck in dem Bilanziersystem 20 zu messen.

Der oder die Drucksensoren haben die Aufgabe, die durch die Volumenverschiebung bedingte Druckänderung in dem okkludierten Flüssigkeitsvolumen zu messen.

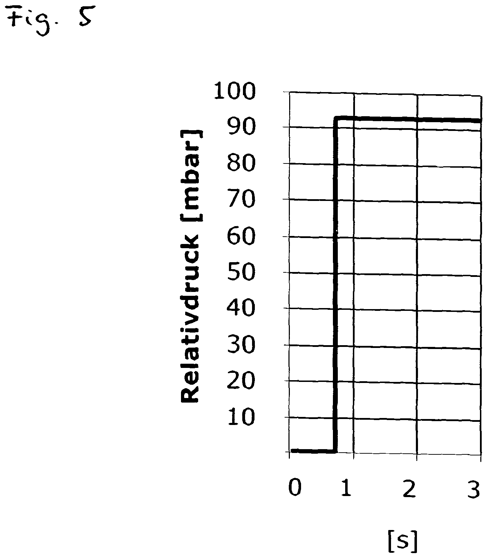

Figur 5 zeigt ein Beispiel für eine Druckmessung vor, während und nach der Volumenzufuhr in den okkludierten Bereich der Kassette als Relativdruck gegenüber dem Atmosphärendruck.

In dem in Figur 5 dargestellten Beispiel ergibt sich durch die Zufuhr eines Flüssigkeitsvolumens in den okkludierten Bereich eine Druckerhöhung von ca. 93 mbar.

Überschreitet der Druck innerhalb der Testzeit eine definierte Grenze, wie z.B. den Wert von 80 mbar innerhalb von 1 s, ist der Rigiditätstest bestanden und die Kassette 1 wird als gut befunden. Wir der Grenzwert gar nicht oder erst nach Ablauf einer Testzeit erreicht oder überschritten, ist der Rigiditätstest nicht bestanden und die Kassette 1 wird verworfen.

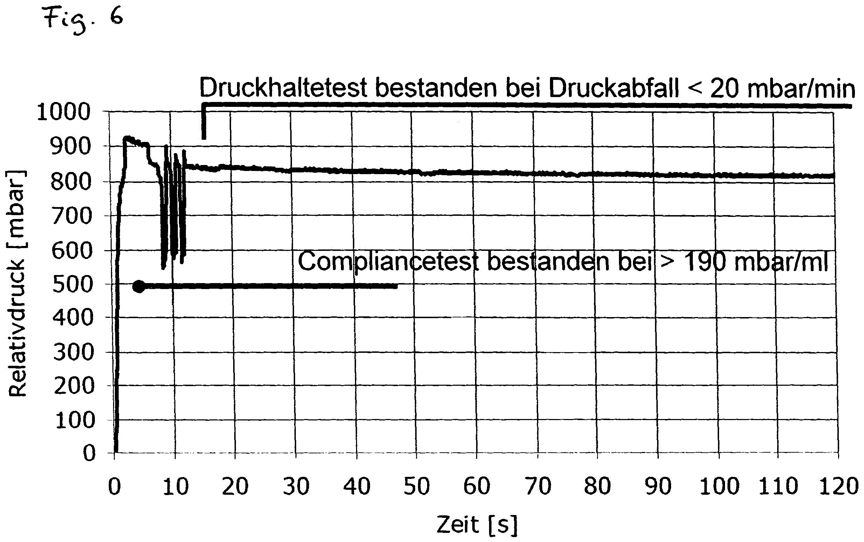

Figur 6 zeigt den zeitlichen Druckverlauf bei der Durchführung eines als Compliancetest genannten Verfahrens gemäß der Erfindung.

Dem in Figur 6 dargestellten Druckverlauf liegt ein Verfahren zugrunde, bei dem zunächst eine Füllung der Kassette 1 vorgenommen wird, so dass der Druck ein bestimmtes Niveau (hier ca. 900 mbar) erreicht.

Sodann wird eine Volumenverschiebung von 1,5 ml in und aus dem geschlossenen System der Kassette 1 vorgenommen.

Wir aus Figur 6 ersichtlich, führt der Abzug von Volumen aus der Kassette 1 zu einer Druckverminderung auf ca. 550 mbar und die Zufuhr von Volumen in die Kassette 1 zu einem Druckanstieg auf ca. 900 mbar. Die durch die Volumenverschiebung bedingte Druckänderung von ca. 350 mbar ist ausreichend, da sie den Grenzwert von 190 mbar/ml übersteigt.

Die vorgenannten Werte sind exemplarisch und nicht beschränkend.

Die wechselnde Volumenverschiebung (Wechsel aus Flüssigkeitszufuhr und - abfuhr) schließt Störeffekte aus.

Der erfindungsgemäße Rigiditätstest wird gemäß Figur 6 mit einem Druckhaltetest kombiniert.

Nach der Durchführung des Rigiditätstests wird kein weiteres Flüssigkeitsvolumen zu- oder abgeführt und der Druck wird weiterhin gemessen.

Liegt der Druckabfall pro Zeit unter einem Grenzwert (hier 20 mbar/min) gilt der Druckhaltetest als bestanden. Der Druckhaltetest geht von einer bestimmten Rigidität der Kassette aus. Ist diese flexibler, muss die Testzeit bzw. der zulässige Grenzwert angepasst werden.

Das erfindungsgemäße Verfahren, d.h. der Rigiditätstest kann vor der Behandlung bzw. von der Inbetriebnahme des Dialysegerätes durchgeführt werden. Er kann auch während der Behandlung durchgeführt werden und durch einen oder mehrere verschiedene Zustände getriggert sein.

## Patentansprüche

1. Verfahren zum Testen der Rigidität eines zur volumetrischen Bilanzierung bestimmten Disposables (1) für ein Blutbehandlungsgerät mit den Schritten: Befüllen des Disposables (1) oder eines Teils von diesem mit einer Flüssigkeit, Einschließen der eingefüllten Flüssigkeit, so dass in dem Disposable (1) oder in dem Teil von diesem ein bestimmtes Volumen der Flüssigkeit vorliegt, Zufuhr und/oder Abfuhr eines bestimmten Flüssigkeitsvolumens in das bzw. aus dem Disposable (1) und Messung der durch die Zufuhr und/oder Abfuhr des Flüssigkeitsvolumens bedingten Druckänderung.

2. Verfahren nach Anspruch 1, **dadurch gekennzeichnet, dass** die Zufuhr und/oder Abfuhr des Flüssigkeitsvolumens mittels einer oder mehrerer Pumpen (30) erfolgt.

3. Verfahren nach Anspruch 2, **dadurch gekennzeichnet, dass** es sich bei der Pumpe (30) um eine Pumpe (30) eines Blutbehandlungsgerätes handelt, wobei vorzugsweise vorgesehen ist, dass es sich bei der Pumpe (30) um die Ultrafiltrationspumpe (30) oder um die Dialysatpumpe handelt.

4. Verfahren nach einem der vorhergehenden Ansprüche, **dadurch gekennzeichnet, dass** das Verfahren vor, nach oder zeitgleich mit einem Druckhaltetest durchgeführt wird.

5. Verfahren nach einem der vorhergehenden Ansprüche, **dadurch gekennzeichnet, dass** die Zufuhr und/oder Abfuhr des Flüssigkeitsvolumens sowie die Messung der Druckänderung mehrfach durchgeführt wird.

6. Verfahren nach einem der vorhergehenden Ansprüche, **dadurch gekennzeichnet, dass** das Einschließen der Flüssigkeit durch das Schließen von Ventilen (X) oder Klemmen erfolgt.

7. Verfahren nach einem der vorhergehenden Ansprüche, **dadurch gekennzeichnet, dass** das Disposable (1) wenigstens zwei Bilanzierkammern aufweist und dass wenigstens ein die Bilanzierkammern verbindender Kanal (4) vorgesehen ist.

8. Verfahren nach einem der vorhergehenden Ansprüche, **dadurch gekennzeichnet, dass** die Druckmessung in oder an der Pumpe (30) oder in oder an einem Bereich des Disposables (1) erfolgt.

9. Blutbehandlungsgerät mit wenigstens einer Aufnahme für ein zur volumetrischen Bilanzierung bestimmtes Disposable (1), wobei das Blutbehandlungsgerät einen oder mehrere Aktoren aufweist, die dazu ausgebildet sind, auf das Disposable (1) einzuwirken, wobei das Blutbehandlungsgerät eine oder mehrere Pumpen (30) aufweist, die dazu ausgebildet sind, ein bestimmtes Flüssigkeitsvolumen in das und/oder aus dem Disposable (1) zu fördern, und wobei das Blutbehandlungsgerät einen oder mehrere Drucksensoren (P) aufweist, die ausgebildet sind, den Druck in der in dem Disposable (1) befindlichen Flüssigkeit zu messen, wobei das Blutbehandlungsgerät wenigstens einen Prozessor aufweist, der die wenigstens eine Pumpe (30) ansteuert und der programmiert ist, ein Verfahren nach einem der Ansprüche 1 bis 8 durchzuführen.

10. Blutbehandlungsgerät nach Anspruch 9, **dadurch gekennzeichnet, dass** es sich bei der Pumpe (30) um die Ultrafiltrationspumpe (30) oder um die Dialysatpumpe des Blutbehandlungsgerätes handelt.

11. Blutbehandlungsgerät nach Anspruch 9 oder 10, **dadurch gekennzeichnet, dass** sich der Drucksensor (P) in oder an der Pumpe (30) oder in oder an dem Disposable (1) befindet.

12. Blutbehandlungsgerät nach einem der Ansprüche 9 bis 11, **dadurch gekennzeichnet, dass** das Blutbehandlungsgerät ein oder mehrere Ventile (X) aufweist, mittels derer ein bestimmtes Flüssigkeitsvolumen in dem Disposable (1) absperrbar ist.

13. Blutbehandlungsgerät nach einem der Ansprüche 9 bis 12, **dadurch gekennzeichnet, dass** das Blutbehandlungsgerät eine Auswerteeinheit aufweist, die ausgebildet ist, die gemessene Druckänderung oder die gemessene Geschwindigkeit der Druckänderung mit einem Grenzwert zu vergleichen.

14. Blutbehandlungsgerät nach Anspruch 13, **dadurch gekennzeichnet, dass** das Blutbehandlungsgerät eine Anzeigeeinheit aufweist, die ausgebildet ist, anzuzeigen, ob die gemessene Druckänderung den Grenzwert übersteigt oder nicht.

15. Blutbehandlungsgerät nach Anspruch 13 oder 14, **dadurch gekennzeichnet, dass** das Blutbehandlungsgerät eine Sperreinheit aufweist, die ausgebildet ist, den Betrieb des Blutbehandlungsgerätes zu verhindern, wenn die gemessene Druckänderung den Grenzwert nicht erreicht oder übersteigt.

## Claims

1. Method for testing the rigidity of a disposable (1) intended for volumetric balancing, for a blood treatment device, comprising the steps of: filling the disposable (1) or a part thereof with a liquid, enclosing the filled liquid, so that a certain volume of the liquid is present in the disposable (1) or the part thereof, supply and/or discharge of a certain volume of liquid into or from the disposable (1) and measuring the pressure change caused by the supply and/or discharge of the volume of liquid.

2. Method according to claim 1, **characterized in that** the supply and/or discharge of the volume of liquid is effected by means of one or multiple pumps (30).

3. Method according to claim 2, **characterized in that** the pump (30) is the pump (30) of a blood treatment device, wherein it is preferably provided that the pump (30) is the ultrafiltration pump (30) or the dialysate pump.

4. Method according to one of the preceding claims, **characterized in that** the method is carried out before, after or simultaneously with a pressure holding test.

5. Method according to one of the preceding claims, **characterized in that** the supply and/or discharge of the volume of liquid as well as the measuring of the pressure change is carried out multiple times.

6. Method according to one of the preceding claims, **characterized in that** the enclosing of the liquid is effected by closing valves (X) or clamps.

7. Method according to one of the preceding claims, **characterized in that** the disposable (1) comprises at least two balancing chambers and that at least one channel (4) connecting the balancing chambers is provided.

8. Method according to one of the preceding claims, **characterized in that** the pressure measuring is carried out in or at the pump (30) or in or at a region of the disposable (1).

9. Blood treatment device having at least one receptacle for a disposable (1) intended for volumetric balancing, wherein the blood treatment device comprises one or multiple actuators, which are configured for acting on the disposable (1), wherein the blood treatment device comprises one or multiple pumps (30) which are configured to convey a certain volume of liquid into and/or out of the disposable (1), and wherein the blood treatment device comprises one or multiple pressure sensors (P) which are configured for measuring the pressure in the liquid located in the disposable (1), wherein the blood treatment device comprises at least one processor which activates the at least one pump (30) and which is programmed to perform a method according to one of claims 1 to 8.

10. Blood treatment device according to claim 9, **characterized in that** the pump (30) is the ultrafiltration pump (30) or the dialysate pump of the blood treatment device.

11. Blood treatment device according to claim 9 or 10, **characterized in that** the pressure sensor (P) is located in or on the pump (30) or in or on the disposable (1).

12. Blood treatment device according to one of claims 9 to 11, **characterized in that** the blood treatment device comprises one or multiple valves (X), by means of which a certain volume of liquid in the disposable (1) can be shut-off.

13. Blood treatment device according to one of claims 9 to 12, **characterized in that** the blood treatment device comprises an evaluation unit which is configured to compare the measured pressure change, or the measured speed of the pressure change, with a threshold value.

14. Blood treatment device according to claim 13, **characterized in that** the blood treatment device comprises a display unit which is configured to display whether or not the measured pressure change exceeds the threshold value.

15. Blood treatment device according 13 or 14, **characterized in that** the blood treatment device comprises a shut-off unit which is configured to prevent the operation of the blood treatment device if the measured pressure change does not reach or exceed the threshold value.

## Revendications

1. Procédé servant à tester la rigidité d'un article à usage unique (1) se destinant à l'équilibrage de la masse volumique pour un appareil de traitement du sang avec les étapes : de remplissage de l'article à usage unique (1) ou d'une partie de celui-ci avec un liquide, de conservation du liquide transvasé de sorte qu'un volume défini du liquide est présent dans l'article à usage unique (1) ou dans la partie de celui-ci, d'amenée et/ou d'évacuation d'un volume de liquide défini dans ou hors de l'article à usage unique (1) et de mesure de la variation de pression due à l'amenée et/ou à l'évacuation du volume de liquide.

2. Procédé selon la revendication 1, **caractérisé en ce que** l'amenée et/ou l'évacuation du volume de liquide sont effectuées au moyen d'une ou de plusieurs pompes (30).

3. Procédé selon la revendication 2, **caractérisé en ce que** la pompe (30) est une pompe (30) d'un appareil de traitement du sang, dans lequel il est prévu de préférence que la pompe (30) est la pompe d'ultrafiltration (30) ou une pompe de dialysat.

4. Procédé selon l'une quelconque des revendications précédentes, **caractérisé en ce que** le procédé est effectué avant, après ou simultanément avec un test de maintien de pression.

5. Procédé selon l'une quelconque des revendications précédentes, **caractérisé en ce que** l'amenée et/ou l'évacuation du volume de liquide ainsi que la mesure de la variation de pression sont effectuées à plusieurs reprises.

6. Procédé selon l'une quelconque des revendications précédentes, **caractérisé en ce que** la conservation du liquide est effectuée par la fermeture de soupapes (X) ou d'attaches.

7. Procédé selon l'une quelconque des revendications précédentes, **caractérisé en ce que** l'article à usage unique (1) présente au moins deux chambres d'équilibrage, et qu'au un canal (4) reliant les chambres d'équilibrage est prévu.

8. Procédé selon l'une quelconque des revendications précédentes, **caractérisé en ce que** la mesure de pression est effectuée dans ou au niveau de la pompe (30) ou dans ou au niveau d'une zone de l'article à usage unique (1).

9. Appareil de traitement du sang avec au moins un logement pour un article à usage unique (1) destiné à l'équilibrage de la masse volumique, dans lequel l'appareil de traitement du sang présente un ou plusieurs actionneurs, qui sont réalisés pour agir sur l'article à usage unique (1), dans lequel l'appareil de traitement du sang présente une ou plusieurs pompes (30), qui sont réalisées pour refouler un volume de liquide défini dans et/ou hors de l'article à usage unique (1) et dans lequel l'appareil de traitement du sang présente un ou plusieurs capteurs de pression (P), qui sont réalisés pour mesurer la pression dans le liquide se trouvant dans l'article à usage unique (1), dans lequel l'appareil de traitement du sang présente au moins un processeur, qui pilote l'au moins une pompe (30) et qui est programmé pour exécuter un procédé selon l'une quelconque des revendications 1 à 8.

10. Appareil de traitement du sang selon la revendication 9, **caractérisé en ce que** la pompe (30) est la pompe d'ultrafiltration (30) ou la pompe de dialysat de l'appareil de traitement du sang.

11. Appareil de traitement du sang selon la revendication 9 ou 10, **caractérisé en ce que** le capteur de pression (P) se trouve dans ou au niveau de la pompe (30) ou dans ou au niveau de l'article à usage unique (1).

12. Appareil de traitement du sang selon l'une quelconque des revendications 9 à 11, **caractérisé en ce que** l'appareil de traitement du sang présente une ou plusieurs soupapes (X), au moyen desquelles un volume de liquide défini peut être bloqué dans l'article à usage unique (1).

13. Appareil de traitement du sang selon l'une quelconque des revendications 9 à 12, **caractérisé en ce que** l'appareil de traitement du sang présente une unité d'évaluation, qui est réalisée pour comparer la variation de pression mesurée ou la vitesse mesurée de la variation de pression à une valeur limite.

14. Appareil de traitement du sang selon la revendication 13, caractérisé en ce l'appareil de traitement du sang présente une unité d'affichage, qui est réalisée pour afficher si la variation de pression mesurée dépasse la valeur limite ou non.

15. Appareil de traitement du sang selon la revendication 13 ou 14, **caractérisé en ce que** l'appareil de traitement du sang présente une unité de fermeture, qui est réalisée pour empêcher le fonctionnement de l'appareil de traitement du sang quand la variation de pression mesurée n'a pas atteint ou dépasse la valeur limite.
